# EUROPEAN PATENT APPLICATION

(11) **EP 4 599 696 A2**
(43) Date of publication of application: **13.08.2025**
(21) Application number: 25184120.1
(22) Date of filing: 09.09.2020
(51) Int. Cl.: A23L 33/125

(54) **COMPOSITION AND METHODS FOR ENHANCING OR PROMOTING A HEALTHY METABOLIC AGING**

(30) Priority: 09.09.2019 EP 19382778
(62) Divisional of application: 20780933.6
(71) Applicant: Servicio Andaluz de Salud, 41071 Sevilla (ES); Euronutra, S.L., 29590 Málaga (ES); Universidad de Málaga, 29071 Málaga (ES)
(72) Inventor: Rodriguez de Fonseca, Fernando, Málaga (ES); Navarro Galera, Juan Antonio, Málaga (ES); Baixeras Llano, Elena, Málaga (ES); Decara del Olmo, Juan Manuel, Málaga (ES); Medina Vera, Dina, Málaga (ES); López Gambero, Antonio Jesús, Málaga (ES); Suarez Perez, Juan, Málaga (ES); Sanjuan Merino, Carlos, Málaga (ES); Rosell del Valle, Cristina, Málaga (ES); Pavón Morón, Francisco Javier, Málaga (ES)
(74) Representative: Hoffmann Eitle

(57) **Abstract**

This invention relates to the delivery of a composition, preferably a pharmaceutical composition, comprising D-pinitol, D-Chiro inositol or myo-inositol or any pharmaceutically acceptable salt thereof, for use in the treatment or prevention of disorders, diseases or conditions responsive to the stimulation of the ghrelin receptor in a subject in need thereof. In particular, for the treatment and/or prevention of disorders responsive to the positive modulation (stimulation) of the ghrelin receptor, such as diabetes, obesity-related disorders, and, most preferably, for treating or preventing age related conditions or diseases such as by promoting appetite, inhibiting insulin secretion and lowering insulin resistance, increasing growth hormone release, enhancing muscle vitality or fragility and treating or preventing sarcopenia by increasing net muscle mass, improving cognition (and/or treating or preventing diseases such as Azlheimer's disease, vascular dementia, Parkinson's Disease, and Huntington's disease) and treating or preventing age related hypertension.

## Description

### Field of the invention

This invention relates to the delivery of a composition, preferably a pharmaceutical composition, comprising D-pinitol, D-Chiro inositol or myo-inositol or any pharmaceutically acceptable salt thereof, for use in the treatment or prevention of disorders, diseases or conditions responsive to the stimulation of the ghrelin receptor in a subject in need thereof.

### Background of the invention

The aging process in humans is associated with physical decline and impairment of metabolic homeostasis (1). While physical decline has a relevant expression on increased fragility and the development of sarcopenia, a generalized loss of muscle mass and muscle cell performance, the dysregulation of the metabolic network leads to age-related increase of obesity, insulin resistance, diabetes and hypertension (2). Ageing impairs the activity of key metabolic signaling pathways and the ensuing metabolic dysregulation results in accelerated ageing. Thus the association of frailty, impaired metabolic homeostasis and hypertension increases vulnerability and limits the quality of life and indeed the life span of the elderly.

A key signaling system impaired in aging is insulin signaling. Age-related impairment in the activity of the insulin signalling pathway results in insulin resistance (1). The ensuing hyperglycemia, as a result of dysregulated glucose clearance, promotes formation of advanced glycation end products (AGEs), which in turn cause tissue damage further exacerbating metabolic dysregulation and accelerating the aging process. A key hepato-muscular loop is profoundly affected by the development of obesity and insulin resistance, where the coupling in between hepatic glucose production and glucose consumption by the muscle is dysregulated as results of the impairment on insulin signaling that leads to overproduction of hepatic glucose, while inhibits its utilization by the muscle, already affected by ageing-associated frailty (1).

Among the different metabolic signalling pathways that control energy homeostasis and muscle vitality, Ghrelin, a peptide hormone produced by specialized cells in the gastrointestinal tract, emerges as a potential target for age-associated metabolic dysregulation and frailty (3-5). Ghrelin is capable of promoting appetite, inhibiting insulin secretion, increasing growth hormone release, enhancing muscle vitality by increasing net muscle mass, and improving cognition (6-9). If we considered that aging produces loss of appetite, excessive insulin secretion an insulin resistance, muscle frailty and cognitive impairment, the physiological effects of Ghrelin is positioned to fight all of these age-associated conditions. Thus, enhancing the release of Ghrelin or the administration of Ghrelin receptor agonists haves been proposed to be used as a method for fighting the above cited age associated conditions (5), including hypertension since decreased Ghrelin levels is positively associated to age-related hypertension (10).

### Brief description of the invention

The present invention refers to a composition, preferably a pharmaceutical composition or a nutraceutical or a food composition, comprising D-pinitol, D-Chiro inositol and/or myo-inositol or any pharmaceutically acceptable salts , esters, tautomers, solvates and hydrates thereof, for use in the treatment or prevention of disorders, diseases or conditions characterized by an impaired ghrelin secretion resulting in active ghrelin blood circulation levels, preferably plasma levels, lower than normal pre-prandial active ghrelin blood circulation, preferably plasma, reference levels in a subject in need thereof. Wherein said disorders, diseases or conditions characterized by an impaired ghrelin secretion resulting in active ghrelin blood circulation levels lower than normal pre-prandial active ghrelin blood circulation reference levels, can be determined by establishing whether such disorders, diseases or conditions are characterized by having altered concentrations of active ghrelin circulation levels as measured with standard and conventional methods such as ELISA or radio immune assays.

In the context of the present invention, "normal pre-prandial reference levels" are understood as pre-prandial reference levels in a healthy subject. Plasma Levels of Ghrelin are discussed, among others, in European Endocrinology, 2015;11(2):90-5 DOI: 10.17925/EE.2015.11.02.90

In the context of the present invention, "active ghrelin" is understood as the acylated (usually n-octanoylated) form of ghrelin. It is generated as a post-translational esterification of a fatty (n-octanoic or, to a lesser extent, n-decanoic) acid on serine residue at position 3 in the secreted Ghrelin. This acylation is necessary for the activity of ghrelin.

Preferably, said disorders, diseases or conditions characterized by an impaired ghrelin secretion resulting in active ghrelin blood circulation levels lower than normal pre-prandial active ghrelin blood circulation reference levels are age related conditions.

In the context of the present invention, "age related conditions" is understood as any physiological change or pathological disorder that is most often seen with increased senescence. Normal physiological changes associated with aging include loss of muscle mass and increased muscle frailty or mild cognitive impairment. Pathological disorders whose prevalence increased exponentially with age include: atherosclerosis, cardiovascular disease, type 2 diabetes, osteoporosis, hypertension, Alzheimer's disease, arthritis, cataracts and cancer.

Preferably, said disorders, diseases or conditions responsive to the modulation of the ghrelin receptor are age related conditions, wherein said composition is preferably for use in enhancing muscle vitality and reducing fragility by increasing net muscle mass.

Preferably, said disorders, diseases or conditions responsive to the modulation of the ghrelin receptor are age related conditions, wherein said composition is preferably for use in the treatment or prevention of sarcopenia.

In the context of the present invention, "sarcopenia" is defined as the loss of skeletal muscle mass and strength as a result of ageing.

Preferably, said disorders, diseases or conditions responsive to the modulation of the ghrelin receptor is hypertension, wherein said composition is for use in the treatment or prevention of hypertension, preferably for use in the treatment or prevention of age-related hypertension.

In the context of the present invention, "age related hypertension" is understood as the increase in systolic blood pressure associated with age derived of the age associated increase in total (and renal vascular) resistance as a consequence of the progressive loss of the viscoelastic properties of conduit vessels, increased atherosclerotic arterial disease, and hypertrophy and sclerosis of muscular arteries and arterioles.

Preferably, said disorders, diseases or conditions characterized by an impaired ghrelin secretion resulting in active ghrelin blood circulation levels lower than normal pre-prandial active ghrelin blood circulation reference levels are age related conditions, wherein said composition is preferably for use in inhibiting insulin secretion and lowering insulin resistance to avoid the progression of the insulin resistance syndrome, a condition where there is a progressive loss of the cellular responses to insulin that results in progressive rise of insulin levels and subsequent endocrine pancreas exhaustion. Diseases that will benefit of a reduction of excess insulin might include diabetes type 2, hypertension, dyslipidaemia, cardiovascular disease, non-alcoholic steatohepathitis and brain insulin resistance associated to Alzheimer's disease.

Preferably, said disorders, diseases or conditions characterized by an impaired ghrelin secretion resulting in active ghrelin blood circulation levels lower than normal pre-prandial active ghrelin blood circulation reference levels are age related conditions, wherein said composition is for use in increasing circulating growth hormone release to compensate the physiological decline in growth hormone release associated with aging, as well as in conditions where we need to increase exercise capacity, muscle mass and bone density because of a low growth hormone secretion such as the infection with Human immunodeficiency virus (HIV).

Preferably, said disorders, diseases or conditions characterized by an impaired ghrelin secretion resulting in active ghrelin blood circulation levels lower than normal pre-prandial active ghrelin blood circulation reference levels are age related conditions, wherein said composition is preferably for use in the treatment or prevention of cognitive impairment or for improving cognition.

In the context of the present invention, "cognitive impairment" is defined by deficits in cognitive abilities (including learning, memory, perception, and problem solving) that are acquired (as opposed to the normal developmental aging associated "cognitive decline"), and may have an underlying brain pathology; "improving cognition" is understood as the amplification or extension of core capacities of the mind through improvement or information processing systems. Cognitive improvement can be achieved by means of interventions that include the administration of a compound included in the present patent.

Preferably, said disorders, diseases or conditions characterized by an impaired ghrelin secretion resulting in active ghrelin blood circulation levels lower than normal pre-prandial active ghrelin blood circulation reference levels are selected from the list consisting of Alzheimer's disease, vascular dementia, Parkinson's Disease, and Huntington's disease.

Preferably, said disorders, diseases or conditions characterized by an impaired ghrelin secretion resulting in active ghrelin blood circulation levels lower than normal pre-prandial active ghrelin blood circulation reference levels are selected from the list consisting of diabetes mellitus, preferably type II diabetes.

Preferably, said composition further comprises a pharmaceutically acceptable carrier.

Preferably, said composition is administered orally or via intragastric to a subject in need thereof.

In addition, the present invention further refers to:
- The non-therapeutic use of a composition comprising D-pinitol, D-Chiro inositol or myo-inositol or any acceptable salt thereof, for promoting appetite.
- The non-therapeutic use of a composition comprising D-pinitol, D-Chiro inositol or myo-inositol or any acceptable salt thereof, for increasing net muscle mass.
- The non-therapeutic use of a composition comprising D-pinitol, D-Chiro inositol or myo-inositol or any acceptable salt thereof, for improving cognition.

### Brief description of the figures

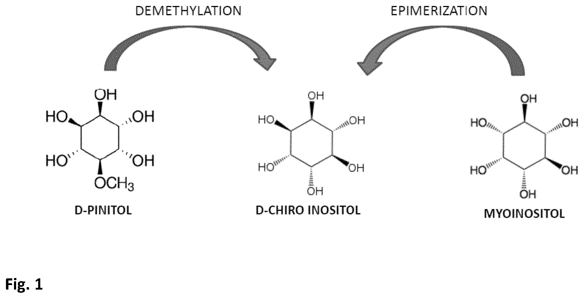

**Figure 1****.** Structure and relationship of D-Pinitol ((1S,2S,4S,5R)-6-methoxycyclohexane-1,2,3,4,5-pentol), D-Chiroinositol (1R,2R,3S,4S,5S,6S)-cyclohexane-1,2,3,4,5,6-hexol and D-myoinositol Myoinositol (1R,2S,3r,4R,5S,6s)-cyclohexane-1,2,3,4,5,6-hexol). Inositols are polialcohols with insulin-mimetic properties. D-Pinitol from natural sources (i.e. carob fruit) can be demethylated in the acid media of the stomach to be converted into D-chiro inositol). In addition, another inositol found in the diet is an isomer of D-chiro inositol that can be converted into D-chiro inositol by means of the enzymatic action of an epimerase.

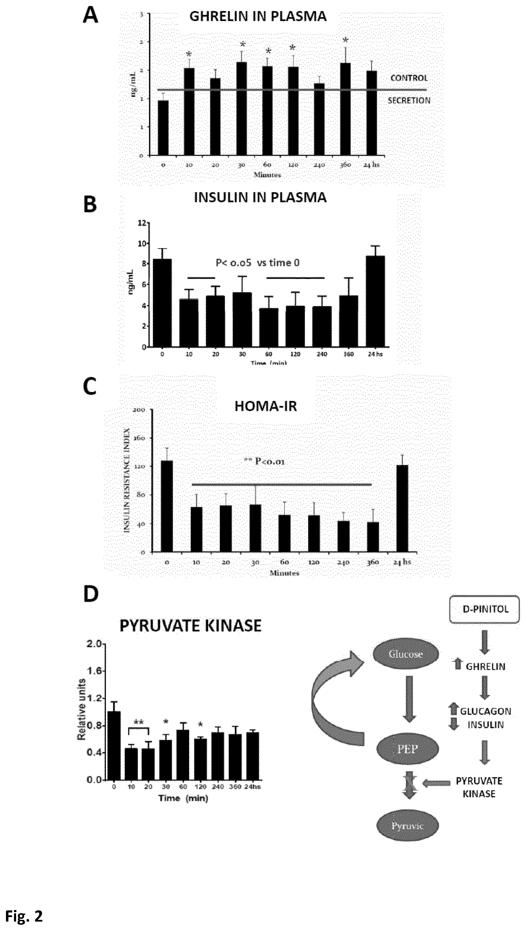

**Figure 2****.** As shown in figure 2, oral administration of Pinitol (100 mg/kg) dissolved in sterile water to adult male Wistar Rats results in a) an increase in circulating plasma Ghrelin concentration that is associated with B) decreased insulin release into the blood circulation, C) reduction of insulin resistance measured by the HOMA index, and D) Inhibition of the expression of Pyruvate Kinase, a key enzyme for diverting phosphoenolpyruvate to glucose production

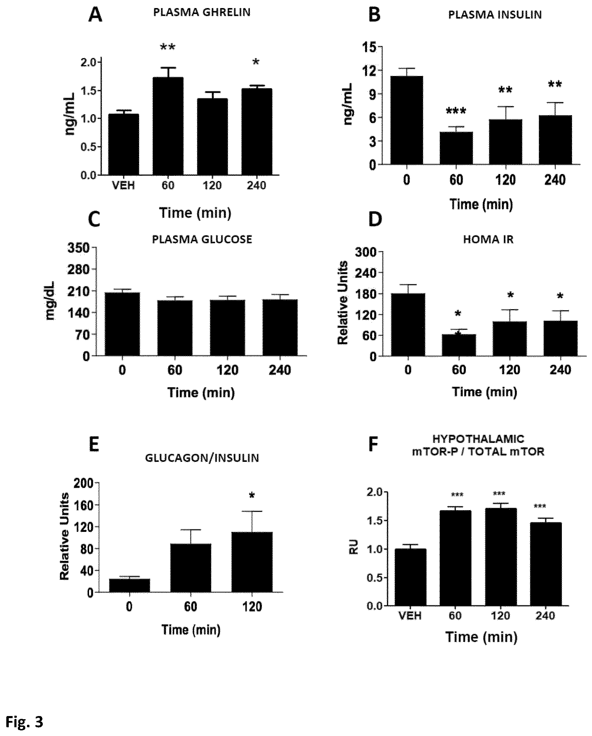

**Figure 3****.** As shown in figure 3, oral administration of Pinitol (500 mg/kg) to adult male Wistar Rats results in A) an increase in circulating plasma Ghrelin concentration that is associated with B) decreased insulin release into the blood circulation, C) maintenance of glucose levels in plasma, D) reduction of insulin resistance measured by the HOMA index, E) Increase in glucagon secretion and F) activation of hypothalamic mTOR signaling in the hypothalamus through its phosphorylation.

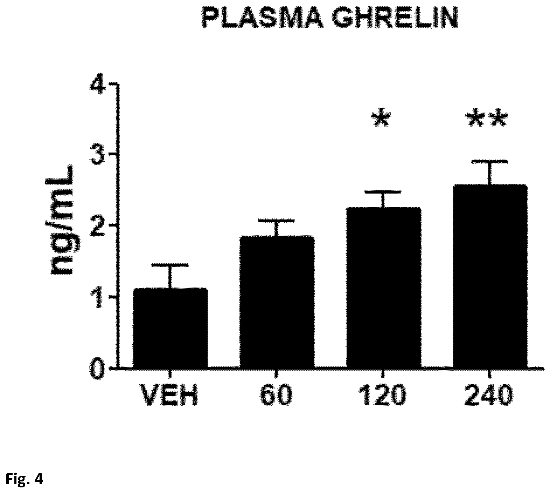

**Figure 4****.** As shown in figure 4, oral administration of D-Chiroinositol (500 mg/kg) enhances Ghrelin secretion as measured through monitorization of circulating plasma Ghrelin concentrations.

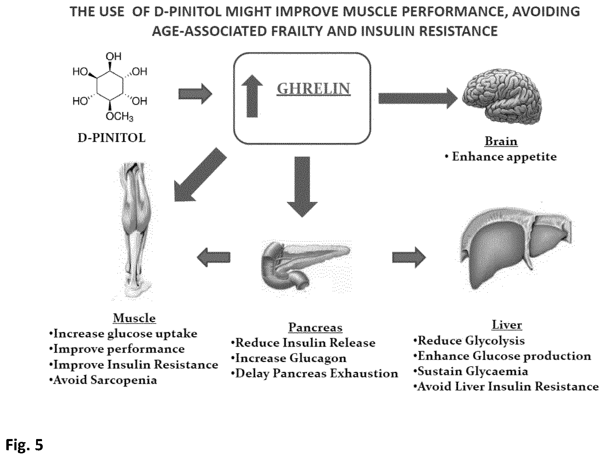

**Figure 5** illustrates a proposed model of action of inositols in metabolic aging. Either D-Pinitol or D-Chiroinositol enhance Ghrelin secretion and promote a metabolic situation characterized by reduced insulin demand from endocrine pancreas, neoglucogenesis in the liver, enhanced muscle use of glucose with associated muscle growth, and enhanced mTOR signaling in the hypothalamus leading to appetite increase. The overall consequences of this unique pharmacological profile might include pancreas protection from exhaustion derived of age-associated insulin resistance and obesity, enhanced muscular vitality (preventing the characteristic sarcopenia and frailty of the elderly) and a better directioning of glucose disposal by the body.

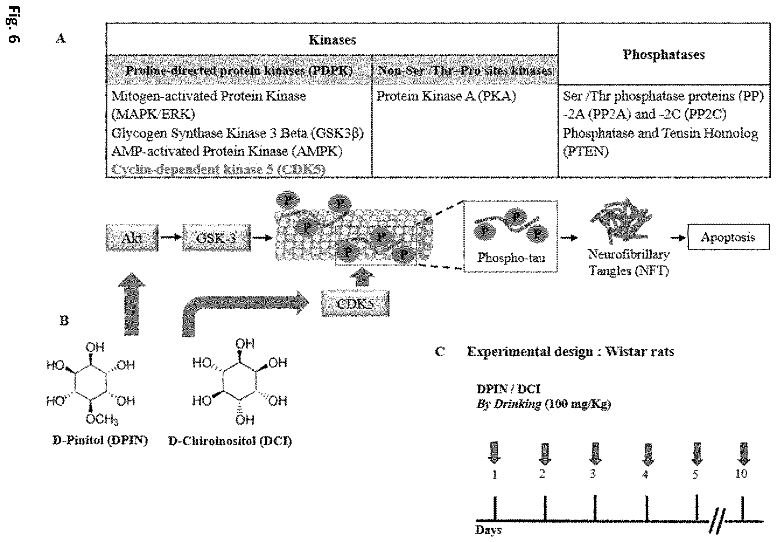

**Figure 6****. (A)** Kinases and Phosphatases analysed in this invention by western blotting to evaluate its activity and/or expression. (C) Schematic representation of tau phosphorylation due to the disfunction of the Akt-GSK3 pathway, or the activation of additional kinases such as protein kinase A (PKA) or cyclin-dependent kinase (CDK5). In physiological conditions, Akt is phosphorylated and, therefore activated. In a pathological condition, such as brain insulin resistance, Akt activates the kinase GSK-3β, which in turn, phosphorylates tau. This can occur when other kinases (PKA, CDK5) are hyperactivated When tau protein is hyperphosphorylated, it dissociates from the microtubule and forms insoluble aggregates called neurofibrillary tangles (NFTs) in neurons and glial cells. Both the destabilization of the microtubule and the aggregation of NFTs leads to cell apoptosis. **(B)** Chemical structure of D-Pinitol (DPIN. DPIN is the 3-O-methyl form of D-chiro-inositol (DCI) and is found as a cyclitol, a cyclic polyol. It is a known anti-diabetic agent isolated from the pulp of the carob fruit (*Ceratonia siliqua*). Schematic timeline of the experimental designs: Chronic drinking administration of DPIN and DCI on Wistar rats were performed on 20 male rats for 10 consecutive days. Plasma and brain samples were collected from both experimental groups. Abbreviations: DPIN, D-Pinitol; DCI, D-Chiro-inositol.

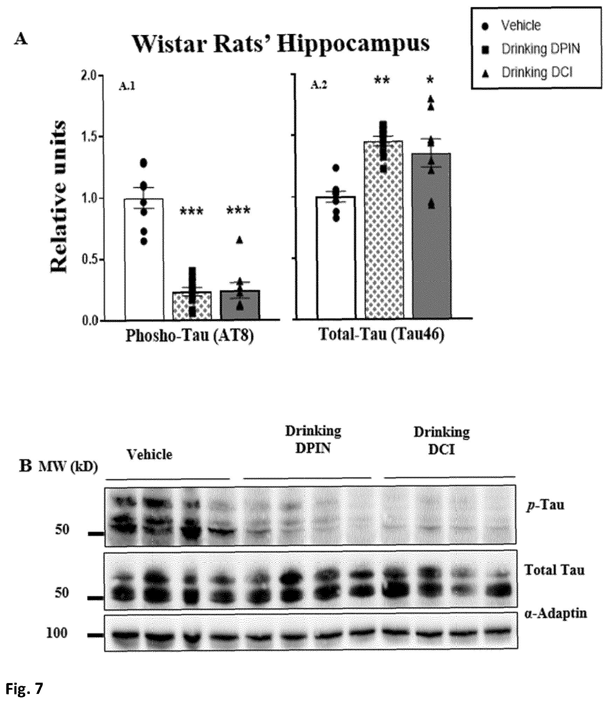

**Figure 7****. Effect of oral administration of DPIN or DCI for 10 days on tau dephosphorization in the hippocampus of Wistar rats.** Bar charts represent the ratio between tau phosphorylation (AT8: Ser202, Thr205) and total tau, and quantity of total tau compared with α-Adaptin on A) Wistar rats, Histograms represent the mean ± S.E.M. (n=8 for Wistar rats). B) Western blot membranes after each antibody incubation (identification target protein right to the band). Molecular weights (MW) are indicated in kilodaltons (kD). The blots shown results from four out of eight independent samples for Wistar rats' groups. The corresponding expression of α-Adaptin is shown as a loading control per lane. One-way ANOVA and Tukey test were performed: (*) P <0.05, (**) P <0.01 and (***) P <0.001 vs Vehicle group.

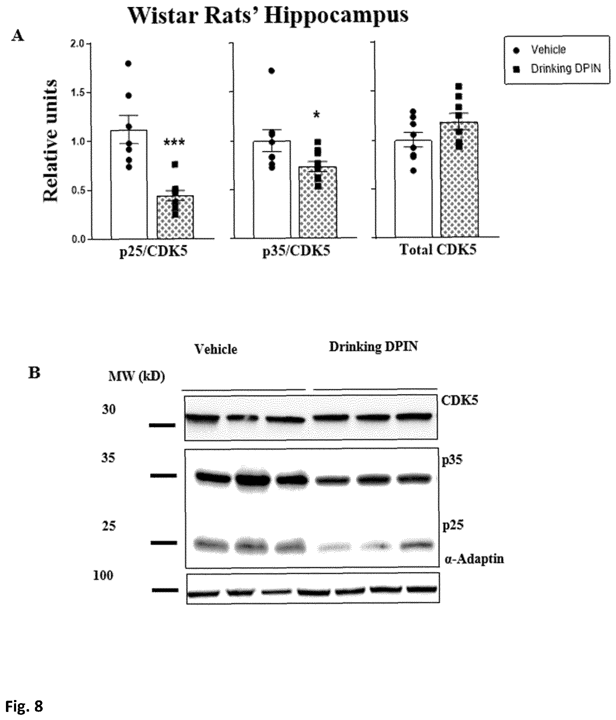

**Figure 8****. Effect of oral administration of DPIN or DCI for 10 days on the tau kinase Cyclin Dependent Kinase 5 (CDK5) activity in the hippocampus of Wistar rats. A)** Bar charts represent the ratio between quantity of the p25, p35 and total CDK5 tau kinase compared with α-Adaptin on Wistar rats. Histograms represent the mean ± S.E.M. (n=8 for Wistar ratsts) **B)** Western blot membranes after each antibody incubation (identification target protein right to the band). Molecular weights (MW) are indicated in kilodaltons (kD). The blots shown results from three out of eight independent samples for Wistar rats' group. The corresponding expression of α-Adaptin is shown as a loading control per lane. Unpaired t-test was performed on Wistar rat's analysis (*) P <0.05, (**) P <0.01 and (***) P <0.001 vs Vehicle group.

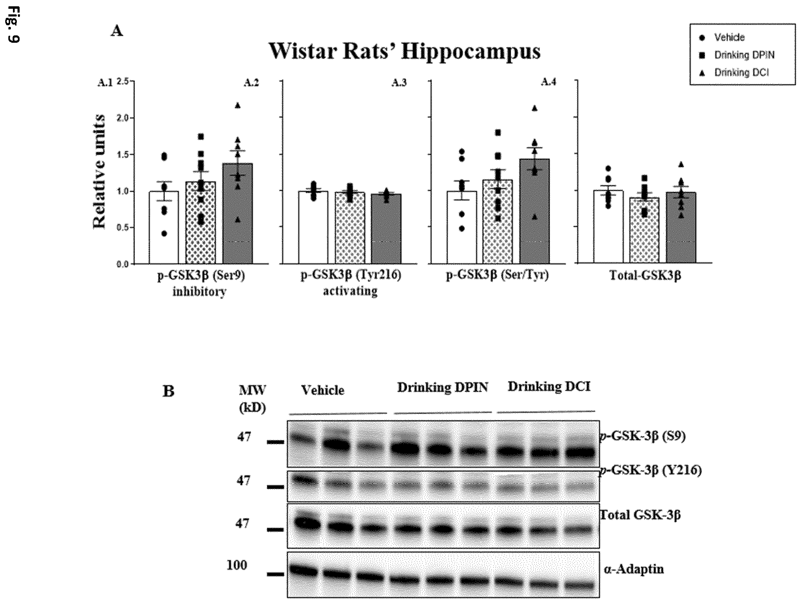

**Figure 9****. Effect of oral administration of DPIN or DCI for 10 days on glycogen synthase kinase-3β (GSK-3 β) phosphorylation in the hippocampus of Wistar rats.** A) Bar charts represent the ratio between GSK-3 β phosphorylation (Ser9/Tyr216) and total GSK3 β, and quantity of total GSK-3 β compared with α-Adaptin on Wistar rats, B) Western blot membranes after each antibody incubation (identification target protein right to the band). Molecular weights (MW) are indicated in kilodaltons (kD). The blots shown results from three out of eight independent samples for Wistar rats' group. The corresponding expression of α-Adaptin is shown as a loading control per lane. Unpaired t-test was performed on Wistar rat's analysis (*) P <0.05, (**) P <0.01 and (***) P <0.001 vs Vehicle group.

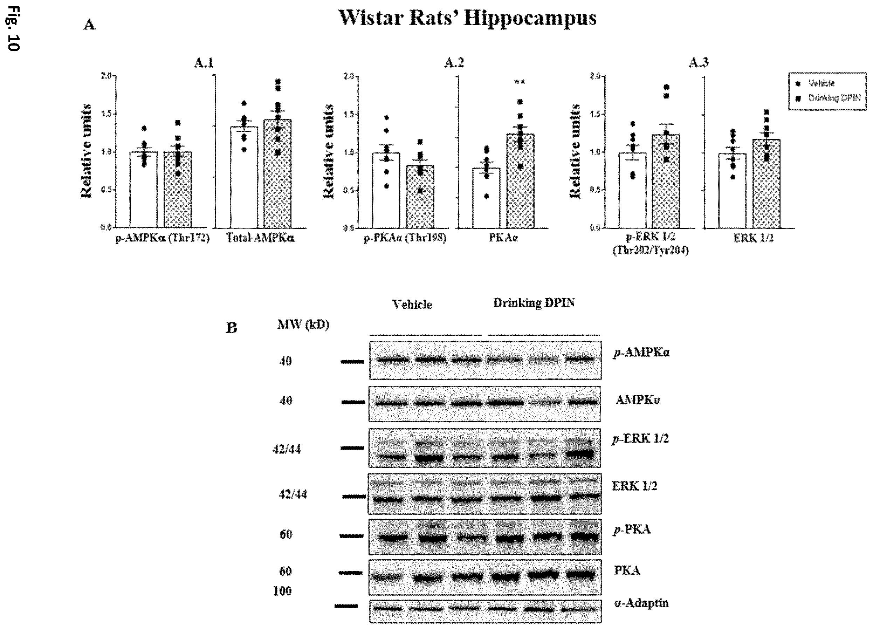

**Figure 10****.** Effect of oral administration of DPIN or DCI for 10 days on other hippocampus's tau kinases: Mitogen-activated Protein Kinase (MAPK), AMP-activated Protein Kinase (AMPK) and Protein Kinase A (PKA) in the hippocampus of Wistar rats. A) Bar charts represent the ratio between quantity of total tau kinases compared with α-Adaptin on Wistar rats. Histograms represent the mean ± S.E.M. (n=8 for Wistar rats). B) Western blot membranes after each antibody incubation (identification target protein right to the band). Molecular weights (MW) are indicated in kilodaltons (kD). The blots shown results from three out of eight independent samples for Wistar rats' group. The corresponding expression of α-Adaptin is shown as a loading control per lane. Unpaired t-test was performed on Wistar rat's analysis (*) P <0.05, (**) P <0.01 and (***) P <0.001 vs Vehicle group.

### Description of the invention

The present invention relates to a method for increasing ghrelin levels as a result of the oral administration of D-pinitol, a natural cyclic polyol derived from plants including the pods of the carob tree, or related inositols such as D-chiro inositol or its epimer myo-inositol (figure 1). Other stereoisomers of inositol could be used, such as cis-inositol, epi-inositol, allo-inositol, muco-inositol, neo-inositol, L-chiro-inositol, acyllo-inositol, or any of the pharmaceutically acceptable salts, esters, tautomers, solvates and hydrates thereof, or any of the combinations thereof. As shown in the examples included throughout the present specification, the oral administration of D-Pinitol (100 mg/kg (Figure 2) or the oral administration of 500 mg/kg of D-Chiro inositol (Figure 3), enhances ghrelin secretion and reduces circulating insulin, and further activates the phosphorylation of mTOR in the hypothalamus, a metabolic sensor needed to increase appetite (figure 3). The net effect is a reduction of the insulin demand, as reflected by the lowering of the insulin resistance index HOMA (Figures 2 & 3), but without leading to hypo- or hyperglycaemia. This is possible thanks to the direct effect of D-pinitol and D-Chiroinositol on the glucose uptake by muscle cells coupled to the net production of glucose by the liver thanks to the inhibition of the pyruvate kinase that redirects glycolisis towards glucose production in the liver (noeglucogenesis) (figure 2). Administration of D-Chiro inositol also enhances ghrelin secretion (figure 4).

In addition, and as proposed in figure 5, the use of D-Pinitol and D-Chiro inositol to promote Ghrelin secretion harmonizes insulin and glucagon secretion, reducing insulin demand, and redirecting glucose production from the liver to its utilization by the muscle. In addition, because of the known actions of ghrelin, it is expected that D-pinitol or related inositols such as D-chiro inositol or its epimer myo-inositol, increase muscle mass and vitality reducing muscle frailty. Since Ghrelin concentrations decline with age (16), these compounds will help promoting a healthy metabolic aging.

On the other hand, the authors of the present invention have surprisingly found a role for D-Pinitol in the prevention of cognitive impairment in aging and in tauopathies. In this sense, the insulin saving properties of D-pinitol and its ability for enhancing Ghrelin secretion, has a pro-cognitive effect that can be used as a preventive strategy for cognitive impairment associated to neurodegeneration. Insulin resistance in the brain is associated to cognitive impairment, in special by modifying hippocampal function. On the other side, Ghrelin is a unique hormone capable of enhancing cognition through its ability of crossing the blood-brain barrier and interaction with the Growth hormone secretagogue receptor. Thus, the novel action described in the present invention might account for the use of D-pinitol administration in aging or in neurodegenerative disorders, specially Alzheimer's Disease to reduce cognitive impairment in early stages of the disease, when symptoms allows diagnosis.

However, recent studies have highlighted the need on focusing in a molecular target that is the best associated factor to the onset of mild cognitive impairment and the development of major dementias: the tau protein. Abnormal phosphorylation or acetylation of tau generates tau protein deposition, causing the appearance of neurofibrillary tangles (NFT) as an hystopathological biomarker of a group of diseases collectively known as tauopathies. The formation of NFT is more strongly correlated with cognitive decline than the distribution of senile plaque, which is formed by polymorphous beta-amyloid (Ax) protein deposits, a pathological hallmark of one of the main dementias, Alzheimer's disease. Tau deposition is thus the crucial element for the cognitive impairment observed in Alzheimer's disease (where β-amyloid deposition is insufficient to generate dementia as well as in the progression towards mild cognitive impairment and the dementia observed in chronic traumatic encephalopathy).

**Thus, tauopathy has been considered as an essential hallmark in neurodegeneration and normal brain aging, and its prevention before the appearance of clinical symptoms an uncovered medical need.** Taking the above into consideration, we examined the effects of the administration of D-Pinitol on the phosphorylation state of Tau, a complex process regulated by multiple proteins (Figure 6) and surprisingly found that the oral administration of D-Pinitol reduced markedly the phosphorylation of Tau (Figure 7), through a mechanism dependent of the reduction on the activity of the cyclin-dependent kinase 5 (Figure 8), one of its major phosphorylation enzymes. D-Pinitol actions were specific since they did not affect other tau-regulatory proteins (Figure 9), providing a unique pharmacological profile for this natural inositol.

Based on the above findings, in the present invention we further propose that the administration of D-Pinitol shall
a) prevent or retard the onset of mild cognitive impairment and its ulterior progression to dementia if a condition is promoting tau hyperphosphorylation and there is an intention of preventing or retarding its clinical appearance. These conditions include Pick disease, progressive supranuclear palsy, corticobasal degeneration, argyrophilic grain disease, globular glial tauopathies, primary age-related tauopathy, which includes neurofibrillary tangle dementia, chronic traumatic encephalopathy (CTE), and aging-related tau astrogliopathy.
b) Treat diagnosed tauopathies, including Pick disease, progressive supranuclear palsy, corticobasal degeneration, argyrophilic grain disease, globular glial tauopathies, primary age-related tauopathy which includes neurofibrillary tangle dementia, chronic traumatic encephalopathy (CTE), and aging-related tau astrogliopathy.

Therefore, the present invention relates to inositols such as D-pinitol, D-chiro inositol and its epimer myo-inositol (from hereinafter compounds of the present invention) for increasing ghrelin levels. Compounds of the present invention are described by the structural formulas identified in figure 1 or by any pharmaceutically acceptable salt derived therefrom. Moreover, the present invention further relates to the compounds of the present invention to reduce the phosphorylation of Tau and thus preventing or slowing the progression of the clinical manifestations of a tauopathy or of mild cognitive impairment in a subject, preferably in a subject before the appearance of such clinical manifestations, more preferably in a human subject.

In the present invention, the term "asymptomatic subject" is understood as a subject that does not show the clinical manifestation of the disease, in particular of a tauopathy.

In the present invention, the term "onset of the clinical manifestation of a tauopathy" is understood as the appearance of cognitive, neuropsychological and/or neurological symptoms and signs, including objective diagnostic procedures (i.e. magnetic resonance imaging, positron emission tomography and cerebrospinal fluid biomarkers) that indicates the diagnostics of a tauopathy.

In the present invention, the term "the onset of the clinical manifestations of mild cognitive impairment" (MCI) is understood as the stage between the expected cognitive decline of normal aging and the more serious decline of dementia. It's characterized by problems with memory, language, thinking or judgment.

In the present invention the term "prevention" means to avoid occurrence of the disease or pathological condition in an individual, particularly when the individual has predisposition for the pathological condition but has not yet been diagnosed. In the present invention, the disease or pathological condition is preferably a "tauopathy".

In the present invention, the term "tauopathy" belongs to a class of neurodegenerative diseases involving the aggregation of tau protein into neurofibrillary or gliofibrillary tangles (NFTs) in the human brain. Tangles are formed by hyperphosphorylation of the microtubule protein known as tau, causing the protein to dissociate from microtubules and form insoluble aggregates. (These aggregations are also called paired helical filaments.) Examples of tauopathies are selected from the list consisting of Pick disease, progressive supranuclear palsy, corticobasal degeneration, argyrophilic grain disease, globular glial tauopathies, primary age-related tauopathy which includes neurofibrillary tangle dementia, chronic traumatic encephalopathy (CTE), and aging-related tau astrogliopathy.

As already indicated, the compounds of the invention are effective for increasing ghrelin levels. They are therefore useful for the treatment and/or prevention of disorders characterized by an impaired ghrelin secretion resulting in active ghrelin blood circulation levels lower than normal pre-prandial active ghrelin blood circulation reference levels, such as in age related conditions, wherein normal physiological changes associated with aging include loss of muscle mass and increased muscle frailty or mild cognitive impairment. Pathological disorders whose prevalence increased exponentially with age further include: atherosclerosis, cardiovascular disease, type 2 diabetes, osteoporosis, hypertension, Alzheimer's disease, arthritis, cataracts and cancer. Preferably, said disorders, diseases or conditions characterized by an impaired ghrelin secretion resulting in active ghrelin blood circulation levels lower than normal pre-prandial active ghrelin blood circulation reference levels is hypertension, preferably age-related hypertension.

Preferably, said disorders, diseases or conditions characterized by an impaired ghrelin secretion resulting in active ghrelin blood circulation levels lower than normal pre-prandial active ghrelin blood circulation reference levels are age related conditions, wherein the compounds of the invention are preferably for use in inhibiting insulin secretion and lowering insulin resistance to avoid the progression of the insulin resistance syndrome, a condition where there is a progressive loss of the cellular responses to insulin that results in progressive rise of insulin levels and subsequent endocrine pancreas exhaustion. Diseases that will benefit of a reduction of excess insulin might include diabetes type 2, hypertension, dyslipidaemia, cardiovascular disease, non-alcoholic steatohepathitis and brain insulin resistance associated to Alzheimer's disease.

Preferably, said disorders, diseases or conditions characterized by an impaired ghrelin secretion resulting in active ghrelin blood circulation levels lower than normal pre-prandial active ghrelin blood circulation reference levels are age related conditions, wherein the compounds of the invention are for use in increasing circulating growth hormone release to compensate the physiological decline in growth hormone release associated with aging, as well as in conditions where we need to increase exercise capacity, muscle mass and bone density because of a low growth hormone secretion such as the infection with Human immunodeficiency virus (HIV).

Preferably, said disorders, diseases or conditions characterized by an impaired ghrelin secretion resulting in active ghrelin blood circulation levels lower than normal pre-prandial active ghrelin blood circulation reference levels are age related conditions, wherein the compounds of the invention are preferably for use in the treatment or prevention of cognitive impairment or for improving cognition.

Also preferably, said disorders, diseases or conditions characterized by an impaired ghrelin secretion resulting in active ghrelin blood circulation levels lower than normal pre-prandial active ghrelin blood circulation reference levels are selected from the list consisting of Alzheimer's disease, vascular dementia, Parkinson's Disease, and Huntington's disease.

Therefore, an aspect of the present invention provides a method for the treatment or prevention of any of the above-mentioned diseases, disorders or conditions in a subject in need thereof, which comprises administering to said subject a therapeutically or prophylactically effective amount of D-pinitol, D-Chiro inositol and/or myo-inositol or any pharmaceutically acceptable salt thereof. The present invention also relates to methods for treating or preventing any of the above-mentioned diseases, disorders or conditions in a subject in need thereof, by administering D-pinitol, D-Chiro inositol and/or myo-inositol or any pharmaceutically acceptable salt thereof in combination with a therapeutically or prophylactically effective amount of another agent known to be useful to treat or prevent any of said conditions or diseases. Another aspect of the present invention provides a pharmaceutical composition comprising a compound having the structural formula D-pinitol, D-Chiro inositol and/or myo-inositol and a pharmaceutically acceptable carrier.

Yet another aspect of the present invention relates to the use of D-pinitol, D-Chiro inositol and/or myo-inositol or any pharmaceutically acceptable salt thereof for the manufacture of a medicament useful for the treatment or prevention, or suppression of a disease characterized by an impaired ghrelin secretion resulting in active ghrelin blood circulation levels lower than normal pre-prandial active ghrelin blood circulation reference levels in a subject in need thereof. In particular for the treatment or prevention, or suppression of any of the above-mentioned diseases, disorders or conditions.

Yet another aspect of the present invention relates to the non-therapeutic use of an amount of D-pinitol, D-Chiro inositol and/or myo-inositol or any salt thereof, for promoting appetite, inhibiting insulin secretion and lowering insulin resistance, increasing growth hormone release, enhancing muscle vitality or fragility by increasing net muscle mass, improving cognition and preventing age related hypertension in a subject in need thereof.

In addition, as already indicated, the compounds of the present invention are useful to markedly reduce the phosphorylation of Tau and thus preventing or slowing the onset or progression of the clinical manifestations of a tauopathy in a subject, preferably in a subject before or after the appearance of such clinical manifestations. Therefore, a further aspect of the present invention provides a method for preventing or slowing the onset or progression of the clinical manifestations of a tauopathy in a subject. More preferably, the present invention provides a method for preventing or slowing the onset of the clinical manifestations of a tauopathy in a subject, that is, a method for preventing or slowing the appearance of such clinical manifestations in said subject, preferably in a healthy subject. In addition, a further aspect of the present invention provides a method for preventing or slowing the onset or progression of the clinical manifestations of mild cognitive impairment in a subject. More preferably, the present invention provides a method for preventing or slowing the onset of the clinical manifestations of mild cognitive impairment in a subject, that is, a method for preventing or slowing the appearance of such clinical manifestations in said subject, preferably in a healthy subject.

Yet another aspect of the present invention relates to the use of D-pinitol, D-Chiro inositol and/or myo-inositol or any pharmaceutically acceptable salt thereof for the manufacture of a medicament useful for preventing or slowing the progression of the clinical manifestations of a tauopathy in a subject, that is, for preventing or slowing the appearance of such clinical manifestations in said subject, preferably in a healthy subject. In addition, a further aspect of the present invention relates to the use of D-pinitol, D-Chiro inositol and/or myo-inositol or any pharmaceutically acceptable salt thereof for the manufacture of a medicament for preventing or slowing the progression of the clinical manifestations of mild cognitive impairment in a subject. More preferably, the present invention relates to the use of D-pinitol, D-Chiro inositol and/or myo-inositol or any pharmaceutically acceptable salt thereof for the manufacture of a medicament useful for preventing or slowing the onset of the clinical manifestations of mild cognitive impairment in a subject, that is, for preventing or slowing the appearance of such clinical manifestations in said subject, preferably in a healthy subject.

In addition to what has been described above, the present invention also encompasses the possibility that a composition of the present invention is in the form of a dietary supplement or nutritional composition that comprises D-pinitol, D-Chiro inositol and/or myo-inositol or any salt thereof. In this sense, in the event that the composition of the invention is formulated as a nutritional composition, said nutritional composition may be a food or be incorporated into a food or food product intended for both human and animal consumption. Thus, in a particular embodiment, the nutritional composition is selected from between a food (which may be a food for specific nutritional purposes or medicinal food) and a nutritional supplement.

In the present invention, the term "nutritional composition" refers to that food, which regardless of providing nutrients to the subject who consumes it, beneficially affects one or more functions of the body, so as to provide better health and wellness.

The term "supplement", synonymous with any of the terms "dietary supplement", "nutritional supplement", "food supplement", or "alimentary supplement" or "alimentary complement" refers to products or preparations whose purpose is to supplement the normal diet consisting of sources of concentrated nutrients or other substances with a nutritional or physiological effect. In the present invention, the "substance" which has a nutritional or physiological effect on the individual when the alimentary complement is ingested are D-pinitol, D-Chiro inositol or myo-inositol or any salt thereof, which are part of any of the compositions of the present invention. The food supplement may be in single or combined form and be marketed in dosage form, i.e. in capsules, pills, tablets and other similar forms, sachets of powder, ampoules of liquids and drop dispensing bottles and other similar forms of liquids and powders designed to be taken in a single amount. The food supplement maybe any plant-derived supplement containing D-Pinitol, including carob fruit-derived syrups.

Preferably, said nutritional compositions or dietary supplements are intended or use for preventing or slowing the progression of the clinical manifestations of a tauopathy in a subject, preferably in a subject before or prior to the appearance of such clinical manifestations, more preferably in a healthy subject. In addition, said nutritional composition or dietary supplements may be further intended or use for preventing or slowing the progression of the clinical manifestations of mild cognitive impairment in a subject. More preferably, for preventing or slowing the onset of the clinical manifestations of mild cognitive impairment in a subject, that is, before or prior to the appearance of such clinical manifestations in said subject, preferably in a healthy subject.

There is a wide range of nutrients and other elements that may be present in alimentary complements including, among others, vitamins, minerals, amino acids, essential fatty acids, fibre, enzymes, plants and plant extracts. Since their role is to complement the supply of nutrients in a diet, they should not be used as a substitute for a balanced diet and intake should not exceed the daily dose expressly recommended by the doctor or nutritionist.

Examples of foods that may comprise the compositions of the invention include, but not limited to, feed, dairy products, vegetable products, meat products, snacks, chocolates, drinks, baby food, cereals, fried foods, industrial bakery products and biscuits. Examples of milk products include, but are not limited to, products derived from fermented milk (for example, but not limited to, yogurt or cheese) or non-fermented milk (for example, but not limited to, ice cream, butter, margarine or whey). The vegetable product is, for example, but not limited to, a cereal in any form of presentation, fermented (for example, soy yogurt, oat yogurt, etc.) or unfermented, and a snack. The beverage may be, but is not limited to, non-fermented milk. In a particular embodiment, the food product or food is selected from the group consisting of fruit or vegetable juices, ice cream, infant formula, milk, yogurt, cheese, fermented milk, powdered milk, lyophilized or air-dried products (suitable for reconstitution with a liquid vehicle), cereals, baked goods, milk-based products, meat products and beverages.

D-pinitol, D-Chiro inositol and/or myo-inositol or any pharmaceutically acceptable salt thereof or ester compounds can be provided in a kit. Such a kit typically contains an active compound of D-pinitol, D-Chiro inositol or myo-inositol in dosage forms for administration. A dosage form contains a sufficient amount of active compound such that a beneficial effect can be obtained when administered to a subject during regular intervals, such as 1, 2, 3, 4, 5 or 6 times a day, during the course of 1 or more days. Preferably, a kit contains instructions indicating the use of the dosage form and the amount of dosage form to be taken over a specified time period.

The term "subject" means a mammal. One embodiment of the term "mammal" is a "human," said human being either male or female. The instant compounds are also useful for treating or preventing age related conditions or diseases such as for promoting appetite, inhibiting insulin secretion and lowering insulin resistance, increasing growth hormone release, enhancing muscle vitality or fragility and treating or preventing sarcopenia by increasing net muscle mass, improving cognition and treating or preventing age related hypertension in cats and dogs. As such, the term "mammal" includes companion animals such as cats and dogs. The term "mammal in need thereof refers to a mammal who is in need of treatment or prophylaxis as determined by a researcher, veterinarian, medical doctor or other clinician.

The term "composition", as in pharmaceutical composition, is intended to encompass a product comprising the active ingredient(s), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier. The term "composition" is also intended to encompass nutraceutical or food compositions. A nutraceutical is a food or food product that provides health and medical benefits, including the prevention and treatment of human ailments.

It will be understood that the compounds of the present invention include hydrates, solvates, polymorphs, crystalline, hydrated crystalline and amorphous forms of the compounds of the present invention, and pharmaceutically acceptable salts thereof. The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, lithium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl- morpholine, N-ethylpiperidine, glucamine, glucosamine, histidine, hydrabamine, isopropylamine, lysine, methylglucamine, moϕholine, piperazine, piperidine, polyamine resins, procaine, purines, theobromine, triethylamine, trimethylamine, tripropylamine, tromethamine, and the like. When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, pamoic, pantothenic, phosphoric, propionic, succinic, sulfuric, tartaric, p-toluenesulfonic acid, trifluoroacetic acid, and the like. Particularly preferred are citric, fumaric, hydrobromic, hydrochloric, maleic, phosphoric, sulfuric, and tartaric acids.

Preferably, and as already indicated throughout the present specification, it will be understood that, as used herein, references to compounds D-pinitol, D-Chiro inositol and myo-inositol are meant to also include the pharmaceutically acceptable salts, such as the hydrochloride salts. The compounds of the present invention are useful in the treatment, control or prevention of diseases, disorders or conditions characterized by an impaired ghrelin secretion resulting in active ghrelin blood circulation levels lower than normal pre-prandial active ghrelin blood circulation reference levels. In particular, the compounds of the present invention are useful in promoting a healthy metabolic aging. Such promotion is carried out by treating or preventing diseases, disorders or conditions that include, but are not limited to, pathological disorders whose prevalence increased exponentially with age such as: atherosclerosis, cardiovascular disease, type 2 diabetes, osteoporosis, hypertension, Alzheimer's disease, arthritis, cataracts and cancer. Preferably, said disorders, diseases or conditions responsive to the modulation of the ghrelin receptor is hypertension, preferably age-related hypertension. Preferably, said disorders, diseases or conditions responsive to the modulation of the ghrelin receptor are age related conditions, wherein the compounds of the invention are preferably for use in inhibiting insulin secretion and lowering insulin resistance to avoid the progression of the insulin resistance syndrome, a condition where there is a progressive loss of the cellular responses to insulin that results in progressive rise of insulin levels and subsequent endocrine pancreas exhaustion. Diseases that will benefit of a reduction of excess insulin might include diabetes type 2, hypertension, dyslipidaemia, cardiovascular disease, non-alcoholic steatohepathitis and brain insulin resistance associated to Alzheimer 's disease. Preferably, said disorders, diseases or conditions responsive to the modulation of the ghrelin receptor are age related conditions, wherein the compounds of the invention are for use in increasing circulating growth hormone release to compensate the physiological decline in growth hormone release associated with aging, as well as in conditions where we need to increase exercise capacity, muscle mass and bone density because of a low growth hormone secretion such as the infection with Human immunodeficiency virus (HIV). Preferably, said disorders, diseases or conditions responsive to the modulation of the ghrelin receptor are age related conditions, wherein the compounds of the invention are preferably for use in the treatment or prevention of cognitive impairment or for improving cognition. Also preferably, said disorders, diseases or conditions responsive to the modulation of the ghrelin are selected from the list consisting of Alzheimer's disease, vascular dementia, Parkinson's Disease, and Huntington's disease.

The compositions of the present invention are thus especially effective for treating Type II diabetes. The compounds or combinations of the present invention are also useful for treating and/or preventing gestational diabetes mellitus. Treatment of diabetes mellitus refers to the administration of a compound or combination of the present invention to treat diabetes. One outcome of treatment may be increasing insulin levels and increasing insulin sensitivity. Another outcome of treatment may be decreasing insulin resistance in a subject with increased insulin resistance. Prevention of diabetes mellitus refers to the administration of a compound or combination of the present invention to prevent the onset of diabetes in a subject at risk thereof.

The terms "administration of and or "administering" a compound should be understood to mean providing a compound of the invention or a prodrug of a compound of the invention to a subject in need of treatment. The administration of the compounds of the present invention in order to practice the present methods of therapy is carried out by administering a therapeutically effective amount of the compound to a subject in need of such treatment or prophylaxis. The need for a prophylactic administration according to the methods of the present invention is determined via the use of well-known risk factors.

The term "therapeutically effective amount" as used herein means the amount of the active compound that will elicit the biological or medical response in a tissue, system, subject, mammal, or human that is being sought by the researcher, veterinarian, medical doctor or other clinician, which includes alleviation of the symptoms of the disorder being treated. The novel methods of treatment of this invention are for disorders known to those skilled in the art. The term "prophylactically effective amount" as used herein means the amount of the active compound that will elicit the biological or medical response in a tissue, system, subject, mammal, or human that is being sought by the researcher, veterinarian, medical doctor or other clinician, to prevent the onset of the disorder in subjects as risk for obesity or the disorder. The therapeutically or prophylactically effective amount, or dosage, of an individual compound is determined, in the final analysis, by the physician in charge of the case, but depends on factors such as the exact disease to be treated, the severity of the disease and other diseases or conditions from which the patient suffers, the chosen route of administration, other drugs and treatments which the patient may concomitantly require, and other factors in the physician's judgement.

Administration and Dose Ranges. Any suitable route of administration may be employed for providing a subject or mammal, especially a human with an effective dosage of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of the invention are administered orally. The effective dosage of active ingredient employed may vary depending on the particular compound employed, the mode of administration, the condition being treated, and the severity of the condition being treated. Such dosage may be ascertained readily by a person skilled in the art.

The magnitude of prophylactic or therapeutic dosage of the compounds of the present invention will, of course, vary depending on the particular compound employed, the mode of administration, the condition being treated, and the severity of the condition being treated. It will also vary according to the age, weight and response of the individual patient. Such dosage may be ascertained readily by a person skilled in the art.

As already stated, compounds of the invention may be used in combination with other drugs that are used in the treatment/prevention/suppression or amelioration of the diseases or conditions for which compounds of the invention are useful. Such other drugs may be administered, by a route and in an amount commonly used therefor, contemporaneously or sequentially with a compound of the invention. When a compound of the invention is used contemporaneously with one or more other drugs, a pharmaceutical composition containing such other drugs in addition to the compound of the invention is preferred.

The compositions of the present invention include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy. In practical use, the compounds of the invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the typical oral dosage unit form, in which case solid pharmaceutical carriers are typically employed. If desired, tablets may be coated by standard aqueous or non-aqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray. The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil. Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of the invention may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

Finally, compounds of the invention may also be administered as nutraceutical or food compositions such as beverages.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), suitable mixtures thereof, and vegetable oils.

The following examples serve to illustrate the present invention but do not limit the same.

### Examples

### Example 1.

### METHODOLOGIY OF EXAMPLE 1

### Animals and ethics statement.

Experimental procedures with animals were carried out in strict accordance with the recommendations in the European Communities directive 2010/63/EU and Spanish legislation (Real Decreto 53/2013, BOE 34/11370-11421, 2013) regulating the care and use of laboratory animals. The protocol was approved by the Ethics Committee for Animal Experiments of the University of Malaga. All studies involving animals were reported in accordance with the ARRIVE guidelines for reporting experiments involving animals (Kilkenny, C., Browne, W. J., Cuthill, I. C., Emerson, M. & Altman, D. G. Improving Bioscience Research Reporting: The ARRIVE Guidelines for Reporting Animal Research. PLoS Biol 8, e1000412, https://doi.org/10.1371/journal.pbio.1000412 (2010). All efforts were made to minimize animal suffering and to reduce the number of animals used. The experiments were performed on 4-to-5-week-old male Wistar rats (Crl:WI; Charles River Laboratories, Barcelona, Spain). The animals were under a standard 12 h light-dark cycle in a room with temperature and humidity control. All the rats were provided with water and commercially available rat standard pellet diet (STD) (3.02 kcal/g with 30 kcal% protein, 55 kcal% carbohydrates and 15 kcal% fat) ad libitum.

### Drug preparation and dose administered.

D-Pinitol (3-0-methyl-D-chiro-inositol) was generously provided by EURONUTRA SL (https://www.euronutra.com/, Málaga, Spain), in the form of crystalline fine powder (lot: PPN-M0201). For acute treatments, D-Pinitol was dissolved in water to be administered by gavage (orally) at different concentrations (100 mg/Kg and 500 mg/Kg), drug was administered at a volume of 1 ml/kg.

### Treatment guidelines.

After 18 hours of fasting, D-Pinitol dissolved in water was administered acutely by gavage (100 mg/kg in a first study, and 500 mg/kg in a subsequent study). After administration, the animals were sacrificed in groups at different times (being time 0 the administration of D-Pinitol). For the first study (100 mg/kg), groups of animals were sacrificed at times: 10, 20, 30, 60, 120, 240 and 360 minutes after administration; for the second study (500 mg/kg), animals were sacrificed at times: 60, 120 and 240 minutes after administration. As a control group, a group was administered only with water (by gavage).

### Sample collection.

Animals were anesthetized with sodium pentobarbital (50 mg/Kg, i.p.), blood, brain and liver samples were collected. Blood was centrifuged (2100 g for 8 min, 4 °C) and the plasma kept for further analysis. Liver and brain samples were flash-frozen in liquid N2, then stored at -80 °C until further analysis.

### Measurement of metabolites in plasma.

The plasma levels of insulin and ghrelin were determined with an enzyme-linked immunosorbent assay (ELISA) method using commercial kits: EMD Millipore Corporation (Billerica, MA, USA) Cat. #EZRMI-13K and Cat. #EZRGRT-91K, respectively. Glucagon levels in plasma was determined with a Glucagon Enzyme Immunoassay (EIA) Kit: Cat. #RAB0202 Sigma-Aldrich (Saint Louis, MO, USA). All serum samples were assayed in duplicate within one assay, and results were expressed in terms of the particular standard hormone.

### RNA isolation and cDNA synthesis.

Total RNA was extracted from tissue portions of liver (100-300 mg) using the Trizol^{®} method according to the manufacturer's instructions (GIBCO BRL Life Technologies). To ensure the purity of the mRNA sequences, RNA samples were isolated with a RNeasy Minelute Cleanup Kit (Qiagen), which included digestion with DNase I column (RNase-free DNase set, Qiagen), according to the manufacturers' instructions. Total RNA was quantified using a spectrophotometer (Nanodrop 1000 Spectrophotometer, Thermo Scientific) to ensure A260/280 ratios of 1.8 to 2.0. Reverse transcription was carried out from 1 µg of RNA using the Transcriptor Reverse Transcriptase kit and random hexamer primers (Transcriptor RT, Roche Diagnostic GmbH). Negative controls included reverse transcription reactions that omitted the reverse transcriptase.

### Real-time Quantitative Polymerase Chain Reaction (qPCR) and Gene Expression Analysis.

Real-time qPCR was performed following the criteria of the MIQE guidelines (Bustin, S. A. et al. The MIQE guidelines: minimum information for publication of quantitative real-time PCR experiments. Clin Chem. 55, 611-622 (2009)). Polymerase chain reactions were carried out on the CFX96 Touch^{™} Real-Time PCR detection system (Bio-Rad, Hercules, CA) for each cDNA template, and amplified in 20 µl reaction volume containing 9 µl of cDNA (diluted 1/100) and 11 µl of master mix containing the primer (TaqMan, Life Technologies). The target rat gen was Pyruvate Kinase Liver/RBC (Pklr) (Supplementary Table). The primer was obtained based on TaqMan^{®} Gene Expression Assays and the FAM^{™} dye label format (Life Technologies). Each reaction was run in duplicate. Cycling parameters were 50 °C for 2 min to deactivate single- and double stranded DNA containing dUTPs, 95 °C for 10 min to activate Taq DNA polymerase followed by 40 cycles at 95 °C for 15 sec for cDNA melting, and 60 °C for 1 minute to allow for annealing and the extension of the primers, during which fluorescence was acquired. Raw fluorescence data were submitted to the online Miner tool (http://www.miner.ewindup.info/) for calculation of Cq and efficiency values for each experimental set (Zhao, S. & Fernald, R. D. Comprehensive Algorithm for Quantitative Real-Time Polymerase Chain Reaction. J Comput Biol. 12, 1047-1064 (2005)). Cq values were converted into relative expression values taking into account amplification efficiencies, inter-run variations, and normalization factors by means of Biogazelle's qbasePLUS software (Biogazelle, Zwijnaarde, Belgium) using at least two reference rat genes: beta Actin (Actb) and Glyceraldehyde-3-phosphate Dehydrogenase (Gapdh) (Supplementary Table). For all reference and target gene studies, two independent biologic samples of each experimental condition were evaluated in technical duplicates. Repeatability between replicates was accepted when the ΔCq value was ≤0.7. Finally, Calibrated Normalized Relative Quantity (CNRQ) values were exported from the qbasePLUS software and investigated statistically.

**Table 1. Primer references for TagMan^{®} Gene Expression Assays (Applied Biosystems).**

| **Gene description** | **Assay ID** | **N° accession GenBank** | **Amplicon Length** |
|---|---|---|---|
| **Target genes** | | | |
| *Pklr* | Rn01455286_m1 | NC_005101.4 | 58 |

| **Reference genes** | | | |
|---|---|---|---|
| Gapdh | Rn01775763_g1 | NC_005103.4 | 174 |
| Actb | Rn00667869_m1 | NM_031144.2 | 91 |

### Protein extraction and Western blot analysis.

Brain extract. Frozen brain samples (17 mg per sample) were homogenized in 1mL of cold RIPA lysis buffer (50 mM Tris-HCl pH 7.4, 150 mM NaCl, 0.5% NaDOC, 1 mM EDTA, 1% Triton, 0.1% SDS, 1 mM Na3VO4, 1 mM NaF) supplemented with a protease cocktail (Roche). The suspension was incubated for 2 hours at 4 °C, followed by centrifugation at 12,000 rpm for 15 minutes at 4 °C. The supernatant was transferred to a new clean centrifuge tube, and Bradford colorimetric method was used to determine the concentration of the total protein. The protein extracts were diluted 1:1 in loading buffer (DTT 2X) and heated for 5 minutes at 99 °C before being subjected to electrophoresis.

Western Blot Analysis. The expression of protein including mTOR (289kDa) and Adaptin (100kDa) were analysed by western blot. The tissue protein (10-15µg) was subjected to electrophoresis on 4-12% Criterion XT Precast Bis-Tris gels (Bio-Rad, Hercules, CA, EE.UU.) for 30 minutes at 80V and 2 h at 150V. Proteins were transferred onto a 0.2 µm nitrocellulose membrane (Bio-Rad, Hercules, CA, EE.UU.) for 1h at 80V by wet transfer equipment. The membrane was washed twice for 5 min in TBST (10 mM Tris-HCl, 150 mM NaCl, 0.1% Tween 20, pH 7.6) and blocked with 5% BSA-TBST for one hour at room temperature on a shaker platform. Subsequently, the membrane was incubated with respective primary antibodies overnight at 4°C diluted in 2% BSA-TBST. Antibodies against p-mTOR (Ser2448) and m-TOR were purchased from Cell Signaling Technology (Danvers, Massachusetts, United States); α-Adaptin from Abcam (Cambridge, United Kingdom). The following day, the membrane was washed three times for 5 min with TBST. An appropriate HRP conjugated rabbit/mouse secondary antibody (Promega, Madison, WI, EE.UU.) was diluted 1:10000 in 2% BSA-TST and incubated with the membrane for 1h shaking at room temperature. Finally, the membrane was washed as above and exposed to chemiluminescent reagent (Santa Cruz, Biotechnology Inc., CA, EE.UU.) for 5 min. Respective membrane bound protein was then visualized by Chemiluminescence (ChemiDoc Imaging System, Bio-Rad). Bands were quantified by densitometric analysis using ImageJ software (Rasband, WS, ImageJ, US National Institutes of Health, Bethesda, MD, USA).

All data are expressed as mean ± SEM. Statistical analysis were conducted in GraphPad Prism, version 8 (GraphPad Software, Inc., La Jolla, CA). One-way analysis of variance (ANOVA) was assessed, followed by a Turkey's Multiple Comparisons Test when appropriate. P values less than 0.05 were considered significant.

### Results of example 1

### 1.1. Results of the oral administration of Pinitol (100 mg/kg) dissolved in sterile water to adult male Wistar Rats

As shown in figure 2, oral administration of Pinitol (100 mg/kg) dissolved in sterile water to adult male Wistar Rats results in a) an increase in circulating plasma Ghrelin concentration that is associated with B) decreased insulin release into the blood circulation, C) reduction of insulin resistance measured by the HOMA index, and D) Inhibition of the expression of Pyruvate Kinase, a key enzyme for diverting phosphoenolpyruvate to glucose production

Furthermore, as shown in figure 3, oral administration of Pinitol (500 mg/kg) to adult male Wistar Rats results in A) an increase in circulating plasma Ghrelin concentration that is associated with B) decreased insulin release into the blood circulation, C) maintenance of glucose levels in plasma, D) reduction of insulin resistance measured by the HOMA index, E) Increase in glucagon secretion and F) activation of hypothalamic mTOR signaling in the hypothalamus through its phosphorylation.

### 1.2. Results of the oral administration of D-Chiroinositol (500 mg/kg) dissolved in sterile water to adult male Wistar Rats

As shown in figure 4, oral administration of D-Chiroinositol (500 mg/kg) enhances Ghrelin secretion as measured through monitorization of circulating plasma Ghrelin concentrations.

### 1.3. Proposed model of action of inositols in metabolic aging

Either D-Pinitol or D-Chiroinositol enhance Ghrelin secretion and promote a metabolic situation characterized by reduced insulin demand from endocrine pancreas, neoglucogenesis in the liver, enhanced muscle use of glucose with associated muscle growth, and enhanced mTOR signaling in the hypothalamus leading to appetite increase. The overall consequences of this unique pharmacological profile might include pancreas protection from exhaustion derived of age-associated insulin resistance and obesity, enhanced muscular vitality (preventing the characteristic sarcopenia and frailty of the elderly) and a better directioning of glucose disposal by the body.

### Example 2

### METHODOLOGY

### Animals and ethics statement

Animal experimental procedures were carried out in accordance with the European Communities directive 2010/63/EU and Spanish legislation (Real Decreto 53/2013). The protocol was approved by the Research and approved by Ethics Committee for Animal Experiments of the University of Malaga, Spain. In accordance with the ARRIVE guidelines, all efforts were made to minimize animal suffering and to reduce the number of animals used per experimental group. Chronic drinking experiments on Wistar rats were performed on 20 male rats. All adult rats, ageing 2 months (≈300 g of body weight), were provided by Charles River Laboratories (Barcelona, Spain). The animals were housed individually under a standard 12 h light-dark cycle in a room with temperature and humidity control. Water and rat chow pellets were provided ad libitum throughout the course of the present study.

### Preparation and administration of inositols

Caromax^{®}-D-Pinitol (3-O-methyl-d-chiro-inositol, DPIN, 98% purity) and Caromax^{®}-D-Chiro-inositol (cis-1,2,3-trans-3,5,6-cyclohexanehexol, DCI) were provided by Euronutra SL (Málaga, Spain). They were dissolved in water to orally daily administration by drinking and/or gavage at dose of 100 mg/Kg of body weight (BW) in a 1 ml/kg of BW volume to Zucker and Wistar rats for 4 weeks (28 days) and 10 days, respectively (Figure 1). During the drinking treatments, the concentration of inositols in the water were updated considering the daily increase of BW of the rats and the possible loss of water by evaporation.

### Protein extraction and Western blot analysis.

- **Brain extract.** Frozen brain samples (17 mg per sample) were homogenized in 1mL of cold RIPA lysis buffer (50 mM Tris-HCl pH 7.4, 150 mM NaCl, 0.5% NaDOC, 1 mM EDTA, 1% Triton, 0.1% SDS, 1 mM Na3VO4, 1 mM NaF) supplemented with a protease cocktail (Hoffmann-Roche). The suspension was incubated for 2 hours at 4 °C, followed by centrifugation at 12,000 rpm for 15 minutes at 4 °C. The supernatant was transferred to a new clean centrifuge tube, and Bradford colorimetric method was used to determine the concentration of the total protein. The protein extracts were diluted 1:1 in loading buffer (DTT 2X) and heated for 5 minutes at 99 °C before being subjected to electrophoresis.
- **Western Blot Analysis.** The tissue protein (10-15 µg) was subjected to electrophoresis on 4-12% Criterion XT Precast Bis-Tris gels (Bio-Rad, USA) for 30 minutes at 80V and 2 h at 150V. Proteins were transferred onto a 0.2 µm nitrocellulose membrane (Bio-Rad, USA) for 1h at 80V by wet transfer equipment. The membrane was washed twice for 5 min in TBST (10 mM Tris-HCl, 150 mM NaCl, 0.1% Tween 20, pH 7.6) and blocked with 2 % Bovine Serum Albumin-Tris Buffered Saline Tween 20 (BSA-TBST) for one hour at room temperature on a shaker platform. Subsequently, the membrane was incubated with respective primary antibodies overnight at 4°C diluted in 2% BSA-TBST (Table 2 for additional information). The following day, the membrane was washed three times for 5 min with TBST. An appropriate HRP conjugated rabbit/mouse secondary antibody (Promega) was diluted 1:10000 in 2% BSA-TST and incubated with the membrane for 1h shaking at room temperature. Finally, the membrane was washed as above and exposed to chemiluminescent reagent (Santa Cruz, Biotechnology Inc.) for 5 min. Stripping/reproving steps were used when necessary. Respective membrane bound protein was then visualized by Chemiluminescence (ChemiDoc Imaging System, Bio-Rad). Bands were quantified by densitometric analysis using ImageJ software (Rasband, WS, ImageJ, US National Institutes of Health, Bethesda, MD, USA). Normalization was performed using a reference protein of the same membrane, the α-Adaptin. The results were expressed as the protein / α-Adaptin ratio or phosphorylated / total protein ratio and normalized to the control group (Y axis represents 'fold mean of the control values').

### Data analysis and statistics

All data are expressed as mean ± SEM. Statistical analysis was undertaken for studies where each group size was of, at least, n = 5. Statistical analysis was conducted in GraphPad Prism, version 8 (GraphPad Software, Inc., San Diego, CA, USA). One- and two-way analysis of variance (ANOVA) was assessed, followed by Turkey's *post hoc* multiple comparisons test. The post hoc tests were conducted only if F in ANOVA achieved a P value less than 0.05 and there was no statistically significant variance inhomogeneity. The analysis of two single groups was performed using Student's unpaired t-test. The results were considered statistically significant at P<0.05.

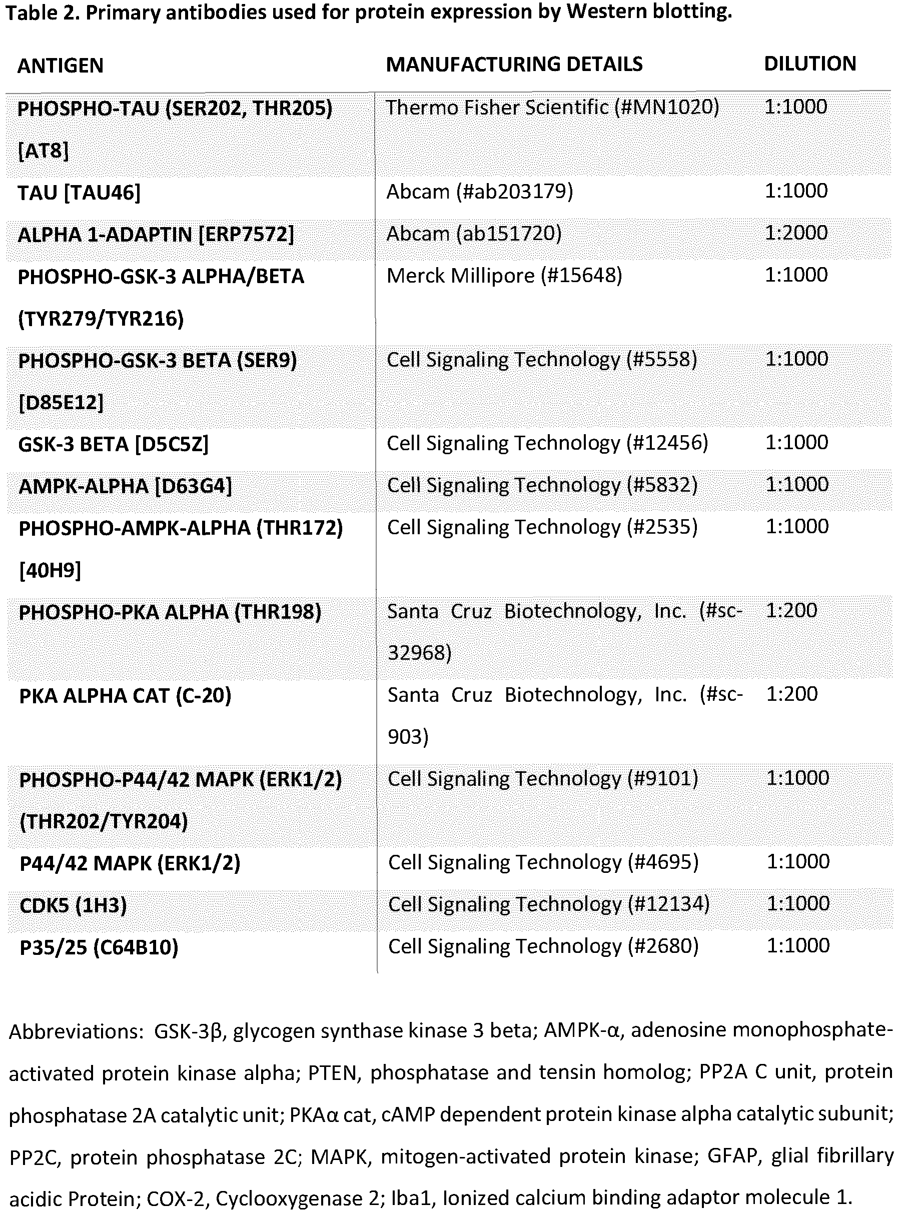

### RESULTS

### 2.1. Effect of D-Pinitol or D-Chiro inositol administrated chronically for 10 days on phosphorylation state of tau protein in the hippocampus of Wistar rats.

Wistar rats were treated orally by drinking DPIN and DCI for 10 days (Figure 6). Here, we analysed the expression and phosphorylation of tau protein, a hallmark of neurodegenerative disorders, such as Alzheimer's disease. For that, we used two specific antibodies: phospho-tau (AT8) antibody which recognizes phosphorylated tau protein in serine 202 and threonine 205. Total Tau (Tau46) antibody recognizes both phosphorylated and non-phosphorylated isoforms. The chronical administration of DPIN and DCI had a statistically significant effect on tau protein dephosphorylation. Quantity of phospho-tau after both compounds has dropped to more than half compared with vehicle group (one-way ANOVA: F_{(2,23)} =49.97, P value <0.0001; Tukey test: P value <0.0001, Figure 7A.1).

We next quantified how much of total tau protein was in the same samples, in order to evaluate whether that drop of phospho-tau was due to less quantity of total tau or not. Significant statistical differences in the relative protein levels of total tau were found after DPIN and DCI treatment (one-way ANOVA: F_{(2,23)} =11.72, P value =0.0003; Tukey test: P value <0.01, Figure 7A.2) reflecting a rise compared with the vehicle group. Therefore, the chronic administration of DPIN and DCI in Wistar rats for 10 days, not only produced a significant reduction of phospho-tau, but also an increase of the total protein amount.

### 2.2. Cyclin Dependent Kinase 5 (CDK5) inhibition after DPIN administration as a putative explanation of hippocampal tau dephosphorylation.

Cyclin-dependent kinase 5 (cdk5) is believed to be involved in the phosphorylation of tau protein. We studied the expression of the protein levels of CDK5, its activator subunit p35 and the truncated form of p35, p25. In Wistar rats, the oral administration, by drinking, of DPIN significantly decreased the amount of the p25 (unpaired t-test: t= 4.869, df=14; P value =0.0002; Figure 8A) as well as the p35 subunit (unpaired t-test: t= 2.245, df=15; P value =0.0402; Figure 8A). The total amount of CDK5 does not vary (unpaired t-test: t= 1.623, df=14; P value =0.1268; Figure 8A). These results indicate a clear inactivation of the CDK5 kinase in Wistar rats after DPIN administration.

### 2.3. Glycogen synthase kinase-3 beta (GSK-3β) activity is not affected in the hippocampus of Wistar after DPIN treatment.

Due to the interaction between the kinase activity of GSK-3β and phosphorylation of the tau protein, we analysed the expression and phosphorylation of this kinase protein. It is well recognized that serine-9 (S9) phosphorylation in GSK-3β causes N-terminal tail to act as pseudo substrate, hindering binding of the actual substrates, thus inhibiting GSK-3. This inhibition of activity is translated into a decrease of tau phosphorylation, as being GSK-3β one of the main tau kinases. In this example, we aimed to study the role of this tau kinase (GSK-3β) in Wistar rats.

Oral administration of either, DPIN or DC,I had no effect either on serine-9 phosphorylation (one-way ANOVA: F_{(2,22)} =1.809, P value =0.1874; Figure 9A.1) or tyrosine-216 (one-way ANOVA: F_{(2,22)} =1.088, P value =0.3543; Figure 9A.2). This means that there is neither enhance nor inhibition of the kinase activity (one-way ANOVA: F_{(2,22)} =2.473, P value =0.1074; Figure 9A.3). In terms of relative protein levels of total GSK-3β, no significant statistical differences were found after the three compounds administration (one-way ANOVA: F_{(2,22)} =0.5308, P value =0.5955; Figure 9A.4). Therefore, in terms of action on GSK-3β in the hippocampus of Wistar rats, neither drinking DPIN or DCI for 10 days does any effect.

### 2.4. AMP-activated Protein Kinase (AMPK), Protein Kinase A (PKA) and Mitogen-activated Protein Kinase (MAPK / ERK1/2) are not related to hippocampal tau dephosphorylation.

Kinase activation and total expression of Mitogen-activated Protein Kinase (MAPK / ERK1/2), AMP-activated Protein Kinase (AMPK) and Protein Kinase A (PKA) were evaluated to analyse whether those kinases were involved in the above tau results. As shown in Figure 10, Wistar rats presents a significant increase of the total amount of PKA with drinking DPIN administration (unpaired t-test: t= 3.795, df=14; P value =0.0020; Figure 10A.2). No other changes are observed among the other kinases.

### References

1.- Bettedi L, Foukas LC. Growth factor, energy and nutrient sensing signaling pathways in metabolic ageing. Biogerontology. 2017 Dec;18(6):913-929.
2.- Soriguer F, et al. Prevalence of diabetes mellitus and impaired glucose regulation in Spain: the Di@bet.es Study. Diabetologia. 2012 Jan;55(1):88-93.
3.- Kojima M, Hosoda H, Date Y, Nakazato M, Matsuo H, Kangawa K (December 1999). "Ghrelin is a growth-hormone-releasing acylated peptide from stomach". Nature. 402 (6762): 656-60
4.- Seim I, Amorim L, Walpole C, Carter S, Chopin LK, Herington AC (January 2010). "Ghrelin gene-related peptides: multifunctional endocrine / autocrine modulators in health and disease". Clinical and Experimental Pharmacology & Physiology. 37 (1): 125-31
5.- Strasser F. Clinical application of ghrelin. Curr Pharm Des. 2012;18(31):4800-12.
6.- Castañeda TR, Tong J, Datta R, Culler M, Tschöp MH (January 2010). "Ghrelin in the regulation of body weight and metabolism". Frontiers in Neuroendocrinology. 31 (1): 44-60.
7.- Inui A, Asakawa A, Bowers CY, Mantovani G, Laviano A, Meguid MM, Fujimiya M (March 2004). "Ghrelin, appetite, and gastric motility: the emerging role of the stomach as an endocrine organ". The FASEB Journal. 18 (3): 439-56.
8.- Alamri BN, Shin K, Chappe V, Anini Y. The role of ghrelin in the regulation of glucose homeostasis. Horm Mol Biol Clin Investig. 2016 Apr 1;26(1):3-11.
9.- Pena-Bello L, Pertega-Diaz S, Outeiriño-Blanco E, Garcia-Buela J, Tovar S, Sangiao-Alvarellos S, Dieguez C, Cordido F. Effect of oral glucose administration on rebound growth hormone release in normal and obese women: the role of adiposity, insulin sensitivity and ghrelin. PLoS One. 2015 Mar 17;10(3):e0121087.
10.- Leinonen T, Antero Kesäniemi Y, Hedberg P, Ukkola O. Serum ghrelin and prediction of metabolic parameters in over 20-year follow-up. Peptides. 2016 Feb;76:51-6.
11.- Martins L, Fernández-Mallo D, Novelle MG, Vázquez MJ, Tena-Sempere M,Nogueiras R, López M, Diéguez C. Hypothalamic mTOR signaling mediates the orexigenic action of ghrelin. PLoS One. 2012;7(10):e46923.
12.- Yada T, Damdindorj B, Rita RS, Kurashina T, Ando A, Taguchi M, Koizumi M, Sone H, Nakata M, Kakei M, Dezaki K. Ghrelin signalling in β-cells regulates insulin secretion and blood glucose. Diabetes Obes Metab. 2014 Sep;16 Suppl 1:111-7.
13.- Kurashina T, Dezaki K, Yoshida M, Sukma Rita R, Ito K, Taguchi M, Miura R, Tominaga M, Ishibashi S, Kakei M, Yada T. The β-cell GHSR and downstream cAMP/TRPM2 signaling account for insulinostatic and glycemic effects of ghrelin. Sci Rep. 2015 Sep 15;5:14041.
14.- Dang NT, Mukai R, Yoshida K, Ashida H. D-pinitol and myo-inositol stimulate translocation of glucose transporter 4 in skeletal muscle of C57BL/6 mice. Biosci Biotechnol Biochem. 2010;74(5):1062-7.
15.- Yap A, Nishiumi S, Yoshida K, Ashida H. Rat L6 myotubes as an in vitro model system to study GLUT4-dependent glucose uptake stimulated by inositol derivatives. Cytotechnology. 2007 Dec;55(2-3):103-8.
16.- Nass R, Farhy LS, Liu J, Pezzoli SS, Johnson ML, Gaylinn BD, Thorner MO. Age-dependent decline in acyl-ghrelin concentrations and reduced association of acyl-ghrelin and growth hormone in healthy older adults. J Clin Endocrinol Metab. 2014 Feb;99(2):602-8.

## Claims

1. A composition, a pharmaceutical composition or a nutraceutical or food composition or dietary supplement, comprising D-Chiro inositol, D-pinitol and/or myo-inositol or any salt thereof, for use in preventing or slowing the onset of the clinical manifestations of mild cognitive impairment or in preventing or slowing the onset of the clinical manifestations of a tauopathy in a subject.

2. The composition for use according to claim 1, wherein the composition is for use in preventing or slowing the onset of the clinical manifestations of mild cognitive impairment in a subject in need thereof.

3. The composition for use according to claim 1, wherein the composition is for use in preventing or slowing the onset of the clinical manifestations of a tauopathy in a subject.

4. The composition for use according to claim 3, wherein the tauopathy is selected from the group consisting of Pick disease, progressive supranuclear palsy, corticobasal degeneration, argyrophilic grain disease, globular glial tauopathies, primary age-related tauopathy which includes neurofibrillary tangle dementia, chronic traumatic encephalopathy (CTE), and aging-related tau astrogliopathy.

5. The composition for use according to any of the precedent claims, wherein the composition comprises D-Chiro inositol.

6. The composition for use according to any of the precedent claims, wherein the composition is a pharmaceutical composition which further comprises a pharmaceutically acceptable carrier.

7. The composition for use according to any of claims 1 to 5, wherein the composition is a dietary supplement.

8. The composition for use according to any of claims 1 to 5, wherein the composition is a nutraceutical composition.

9. The composition for use according to any of claims 1 to 8, wherein said composition is administered orally or via intragastric.

10. The composition for use according to any of claims 1 to 9, wherein the composition is administered to a healthy subject that does not suffer any clinical manifestations of a tauopathy or of mild cognitive impairment.
